# EUROPEAN PATENT APPLICATION

(11) **EP 3 998 325 A1**
(43) Date of publication of application: **18.05.2022**
(21) Application number: 20841253.6
(22) Date of filing: 15.05.2020
(51) Int. Cl.: C09K 15/06, G01N 33/72

(54) **AGENT FOR PREVENTION OF DECOMPOSITION OF BIOPYRRIN**

(30) Priority: 12.07.2019 JP 2019130009
(71) Applicant: Resvo Inc., Kawasaki-shi, Kanagawa 210-0007 (JP)
(72) Inventor: OHNISHI Arata, Kawasaki-shi, Kanagawa 210-0007 (JP)
(74) Representative: Maikowski & Ninnemann Patentanwälte Partnerschaft mbB
(86) International application number: PCT/JP2020/019462
(87) International publication number: WO 2021/010010

(57) **Abstract**

The inventors discovered that antioxidant substances having enediol structure can prevent the decomposition of biopyrrin. Therefore, the invention provides agents for preventing decomposition of biopyrrin, comprising an antioxidant substance having enediol structure, or a salt, an ester or a glycoside thereof, or a solvate, a stereoisomer or a tautomer thereof. In a biological sample to which the agent for preventing decomposition of the invention is added, the decomposition of biopyrrin can be prevented at ambient temperature or lower for a long term.

## Description

### Technical Field

The invention relates to an agent for preventing decomposition of biopyrrin, a biological sample to which the agent for preventing decomposition of the invention is added, and a method of using an agent for preventing decomposition of biopyrrin, for example, a method of preventing decomposition of biopyrrin in a biological sample by using the agent for preventing decomposition of biopyrrin, a method of storing a biological sample by using the agent for preventing decomposition of biopyrrin, a method of measuring a concentration of biopyrrin in a biological sample by using the agent for preventing decomposition of biopyrrin, and a method of evaluating an oxidative stress state by using the agent for preventing decomposition of biopyrrin.

### Background Art

Biopyrrin is an oxidative decomposition product produced when bilirubin reacts with reactive oxygen species in vivo. It is suggested that in vivo biopyrrin is useful as a marker for oxidative stress or psychiatric disorders (Non-patent Literatures 1 to 9), and some methods and devices for measuring in vivo biopyrrin have been developed (Patent Literature 1 and Non-patent Literature 8). However, biopyrrin decomposes quickly, causing problems and difficulties in transportation and storage. In fact, when a urine sample is collected from a patient, biopyrrin in the sample decomposes during the transportation or storage of the sample, and the biopyrrin concentration in the sample cannot be measured accurately. For this reason, a diagnosis method utilizing in vivo biopyrrin as a marker has not been put into practical use yet. Accordingly, an agent for preventing decomposition of biopyrrin has been required.

### Citation List

### Patent Literature

Patent Literature 1: Japanese Patent Laid-Open No. 2007-218593

### Non-patent Literature

Non-patent Literature 1: Masayo Matsuzaki et al., "Effects of lifestyle factors on urinary oxidative stress and serum antioxidant markers in pregnant Japanese women: A cohort study", BioScience Trends. 2014; 8(3):176-184
Non-patent Literature 2: Tsuyoshi Miyaoka et al., "Urinary excretion of biopyrrins, oxidative metabolites of bilirubin, increases in patients with psychiatric disorders", European Neuropsychopharmacology 15 (2005) 249-252
Non-patent Literature 3: Tsuyoshi Miyaoka et al., "Analysis of oxidative stress expressed by urinary level of biopyrrins and 8-hydroxydeoxyguanosine in patients with chronic schizophrenia", Psychiatry and Clinical Neurosciences 2015; 69: 693-698
Non-patent Literature 4: Tomoya Miyashita et al., "Social stress increases biopyrrins, oxidative metabolites of bilirubin, in mouse urine", Biochemical and Biophysical Research Communications 349 (2006) 775-780
Non-patent Literature 5: Tokio Yamaguchi et al., "Psychological stress increases bilirubin metabolites in human urine", Biochemical and Biophysical Research Communications 293 (2002) 517-520
Non-patent Literature 6: Rei Yasukawa et al., "Increased urinary excretion of biopyrrins, oxidative metabolites of bilirubin, in patients with schizophrenia", Psychiatry Research 153 (2007) 203-207
Non-patent Literature 7: Institute for Environmental Sciences, University of Shizuoka, Kayoko Shimoi et al., "Knowing the degree of stress by urine components", Shizuoka Chubu City Area Industry-Academia-Government Cooperation Promotion Program, Foods Science Hills, 2006, Research Results
Non-patent Literature 8: Izuru shioji et al., "Biopyrrin", Rinshoukennsa, Vol.45, no.3, 2011, p.265-269
Non-patent Literature 9: Tokio Yamaguchi et al., "Low molecule antioxidant substances (Principally antioxidant effects of bilirubin)", J Anal Bio-Sci (Seibutsu Shiryo Bunseki), Vol. 32, No.4 (2009), p.281-288

### Summary

### Technical Problem

The object of the invention is to provide an agent for preventing decomposition of biopyrrin. Solution to Problem

Biopyrrin is a decomposition product of bilirubin decomposed by reactive oxygen species. The inventors considered that biopyrrin could be affected by reactive oxygen species, and studied whether or not the decomposition of biopyrrin is prevented by using antioxidant substances in some kinds. Thereby, the inventors discovered that an antioxidant substance having enediol structure can prevent the decomposition of biopyrrin (See Example 1).

Accordingly, the invention provides an agent for preventing decomposition of biopyrrin, comprising an antioxidant substance having enediol structure, or a salt, an ester or a glycoside thereof, or a solvate, a stereoisomer or a tautomer thereof. In a specific embodiment, the antioxidant substance having enediol structure is a reductone. In a further specific embodiment, the reductone is a compound having 3,4-dihydroxy-furan-2(5H)-one structure. In a further specific embodiment, the compound having 3,4-dihydroxy-furan-2(5H)-one structure is a compound of formula (V): 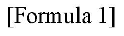 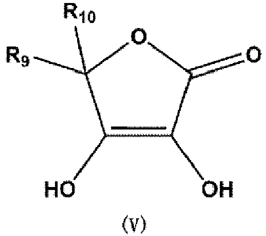 wherein R⁹ and R¹⁰ are, each independently, hydrogen; alkyl; or alkyl substituted with one or more of the same or different substituents selected from a group consisting of hydroxy, carboxy, alkoxy, alkoxycarbonyl, oxo, acyl and acyloxy. In a further specific embodiment, the compound of formula (V) is ascorbic acid.

The invention also provides a biological sample to which added is the agent for preventing decomposition according to the invention. In a specific embodiment, the biological sample is urine. In a specific embodiment, the antioxidant substance having enediol structure comprised in the agent for preventing decomposition is present in the biological sample at a concentration within a range of about 1 mM to about 20 mM. In a further specific embodiment, the antioxidant substance having enediol structure comprised in the agent for preventing decomposition is present in the biological sample at a concentration of about 10 mM.

The invention further provides a method of preventing decomposition of biopyrrin in a biological sample, comprising a step of adding the agent for preventing decomposition of the invention to the biological sample.

The invention further provides a method of storing a biological sample, comprising steps of: (a) adding the agent for preventing decomposition of the invention to the biological sample; and (b) storing the biological sample at ambient temperature or lower.

The invention further provides a method of measuring a concentration of biopyrrin in a biological sample, comprising steps of: (a) adding the agent for preventing decomposition of the invention to the biological sample; and (b) measuring the concentration of biopyrrin in the biological sample. In one embodiment, the method further comprises a step of storing the biological sample at ambient temperature or lower after step (a).

The invention further provides a method of obtaining data for assessing an oxidative stress state in a subject, comprising steps of : (a) adding the agent for preventing decomposition of the invention to a biological sample obtained from the subject; and (b) measuring a concentration of biopyrrin in the biological sample. Here, when the measured concentration is higher than a concentration of biopyrrin in a reference sample, the concentration data indicates that the subject is under oxidative stress state. In one embodiment, the method further comprises a step of storing the biological sample at ambient temperature or lower after step (a).

In one embodiment, the biological sample in said methods is urine. In a specific embodiment, the antioxidant substance having enediol structure comprised in the agent for preventing decomposition is added to the biological sample to a concentration within a range of about 1 mM to about 20 mM. In a further specific embodiment, the antioxidant substance having enediol structure comprised in the agent for preventing decomposition is added to the biological sample to a concentration of about 10 mM.

### Effects of Invention

The agent for preventing decomposition of the invention can prevent the decomposition of biopyrrin.

### Brief Description of Drawings

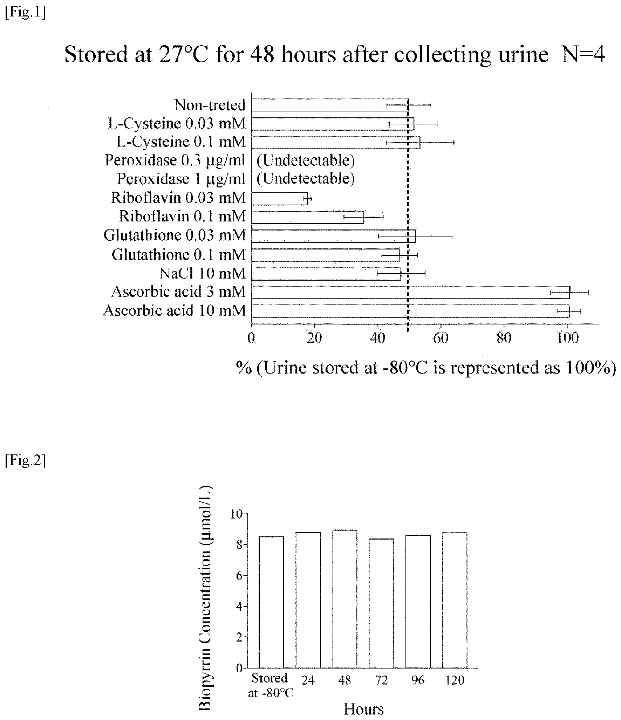

Figure 1 shows results of decomposition prevention test of biopyrrin in urine sample using a variety of antioxidant substances. Each of a variety of antioxidant substances was added to urine samples, and the samples were incubated for 48 hours at 27 °C. Each concentrations was calibrated with a concentration of biopyrrin in a standard sample (antioxidant substance-free, stored at -80°C) as 100%.
Figure 2 shows chronological changes in the concentration of biopyrrin in urine sample using the agent for preventing decomposition of the invention. Ascorbic acid was added to each of urine samples to a final concentration of 10 mM, and the samples were stored at 4 °C. The concentrations of biopyrrin in urine samples were measured after 24 hours, 48 hours, 72 hours, 96 hours and 120 hours after the addition.

### Description of Embodiments

### (1. Definitions)

As used herein, the term "biopyrrin" has a meaning in the broadest sense in the art of the invention, and in general, means a decomposition product of bilirubin decomposed by reactive oxygen species in vivo. It is known that biopyrrin is useful as an oxidative stress marker, a marker for at-risk mental state (ARMS) and a marker for psychiatric disorders. Since biopyrrin is hydrophilic and is excreted in urine, a biopyrrin level in urine can reflect an amount of reactive oxygen species in the body.

As used herein, the term "agent for preventing decomposition" has a meaning in the broadest sense in the art of the invention, and in general, means an agent for preventing decomposition of a substance. An agent for preventing decomposition can also be called a preservative. In one embodiment, an agent for preventing decomposition is an agent for preventing oxidation (an antioxidant or oxidation inhibitor).

As used herein, the term "antioxidant substance" has a meaning in the broadest sense in the art of the invention, and in general, means a compound for inhibiting oxidation reaction mediated by oxygen in the air, oxygen dissolved in a liquid, reactive oxygen species in the body, and the like.

As used herein, the term "salt" has a meaning in the broadest sense in the art of the invention, and in general, it can be formed by using an acid(s) or base(s) selected from a group including inorganic acids or inorganic bases, or organic acids or organic bases. Examples of salts include, but are not limited to, metal salts formed with aluminum, calcium, lithium, magnesium, potassium, sodium, zinc and the like, or organic salts formed with lysine, N,N'-dibenzylethylenediamine, chloroprocaine, choline, diethanolamine, ethylenediamine, meglumine (N-methylglucamine), procaine and the like. Also, salts may contain acid-addition salts and base-addition salts.

As used herein, the term "glycoside" has a meaning in the broadest sense in the art of the invention, and in general, means a sugar bound to a non-sugar part (aglycone) via a glycoside bond. Glycosides are also called glycoside. Glycosides include O-glycosides. As used herein, the term "O-glycoside" has a meaning in the broadest sense in the art of the invention, and in general, means a glycoside in which hydroxy of aglycone is fused to hemiacetalic hydroxy of a cyclic sugar.

As used herein, the term "ester" has a meaning in the broadest sense in the art of the invention, and in general, means a compound formed by removing water from an acid and an alcohol. Esters include carboxylic acid esters, phosphoric acid esters (for example, phosphoric acid monoesters), salts of phosphoric acid esters, or the like.

As used herein, the term "solvate" has a meaning in the broadest sense in the art of the invention, and in general, means a solvent-containing compound that is formed by association of one or a plurality of solvent molecules to the compounds of the present invention. Solvates include, for example, monosolvates, disolvates, trisolvates, and tetrasolvates. When solvates are water, solvates are hydrates.

As used herein, the term "stereoisomer" has a meaning in the broadest sense in the art of the invention. Stereoisomers include diastereomers (cis-trans isomers etc.) and enantiomers.

As used herein, the term "tautomer" has a meaning in the broadest sense in the art of the invention. Tautomers include keto-enol tautomers.

As used herein, the term "reductone" has a meaning in the broadest sense in the art of the invention, and in general, means a compound having a carbonyl group adjacent to enediol, that is, a compound having -C(OH)=C(OH)-C(=O)- group. Reductones may be straight-chain compounds or cyclic compounds.

As used herein, the term "biological sample" has a meaning in the broadest sense in the art of the invention, and in general, means a sample such as urine, blood (serum, plasma or whole blood), tissues, or cells.

As used herein, the term "about" has a meaning in the broadest sense in the art of the invention, and in general, means an inclusion of an acceptable margin of error for an indicated numeric value. The term "about", for example, means that there is a fluctuation of ± 0.1 to 20%, ± 0.1 to 10%, ± 0.1 to 5%, ± 0.1 to 1.0%, or ± 0.1 to 0.5% of a given value or a value range.

As used herein, the term "oxidative stress" or "oxidative stress state" has a meaning in the broadest sense in the art of the invention, and in general, means an imbalance state between production of reactive oxygen species and their elimination in the body.

As used herein, the term "alkyl" means a monovalent group formed by removing a hydrogen atom from aliphatic saturated hydrocarbon. Alkyl has, for example, 1 to 20 carbon atoms, and typically has 1 to 10, 1 to 8, 1 to 6, 1 to 5, 1 to 4, 1 to 3, 1 to 2, or 2 to 6 carbon atoms. Alkyl may be a straight-chain or may be branched. Examples of alkyl include, but are not limited to, methyl, ethyl, n-propyl, isopropyl, n-butyl, isobutyl, t-butyl, n-pentyl, n-hexyl, n-decyl, n-pentadecyl, n-hexadecyl, n-heptadecyl, n-octadecyl and the like. Alkyl may be substituted with one or more (for example, one, two, three, four, five or more) of the same or different substituents.

As used herein, the term "alkenyl" means a monovalent group formed by removing a hydrogen atom from aliphatic unsaturated hydrocarbon that has at least one double bond. Alkenyl has, for example, 2 to 20 carbon atoms, and typically has, 2 to 10, 2 to 8, 2 to 6, 2 to 5, 2 to 4, 2 to 3, 3 to 6, 3 to 8, 4 to 6, 4 to 7, or 4 to 8 carbon atoms. Alkenyl may be a straight-chain or may be branched. Examples of alkenyl include, but are not limited to, vinyl (-CH=CH₂), allyl (-CH₂CH=CH₂), 1,3-butadienyl (-CH=CH-CH=CH₂) and the like. Alkenyl may be substituted with one or more (for example, one, two, three, four, five or more) of the same or different substituents.

As used herein, the term "alkynyl" means a monovalent group formed by removing a hydrogen atom from aliphatic unsaturated hydrocarbon that has at least one triple bond. Alkynyl has, for example, 2 to 20 carbon atoms, and typically has 2 to 10, 2 to 8, 2 to 6, 2 to 5, 2 to 4, 2 to 3, 3 to 6, 4 to 6, 4 to 7, or 4 to 8 carbon atoms. Alkynyl may be a straight-chain or may be branched. Examples of alkynyl include, but are not limited to, ethynyl (-C=CH), -CH-C=CH and the like. Alkynyl may be substituted with one or more (for example, one, two, three, four, five or more) of the same or different substituents.

As used herein, the term "alkoxy" means -OR, where R is any hydrocarbon group, for example, alkyl, alkenyl, alkynyl, a non-aromatic carbocyclic group, a non-aromatic heterocyclic group, an aromatic carbocyclic group, an aromatic heterocyclic group or the like which is unsubstituted or substituted with one or more (for example, one, two, three, four, five or more) of the same or different substituents. Examples of alkoxy include, but are not limited to, methoxy, ethoxy, propoxy, butoxy and the like.

As used herein, the term "alkoxycarbonyl" means -CO-alkoxy, that is, -COOR. R is as described in the item "alkoxy".

As used herein, the term "hydroxy" means -OH.

As used herein, the term "hydroxyalkyl" means an alkyl group in which at least one (for example, one, two, three, four or more) of hydrogen atoms is substituted with -OH. Examples of hydroxyalkyl include, but are not limited to, 1-hydroxyethyl, 2-hydroxyethyl, 1,1-dihydroxyethyl, 1,2-dihydroxyethyl, 2,2-dihydroxyethyl, 1,1,2-trihydroxyethyl, 1,2,2-trihydroxyethyl, 2,2,2-trihydroxyethyl, 1,2,3-trihydroxypropyl and the like.

As used herein, the term "carboxy" means -COOH.

As used herein, the term "acyl" means a group represented by -CO-R', where R' is any hydrocarbon group, for example, alkyl, alkenyl, alkynyl, a non-aromatic carbocyclic group, a non-aromatic heterocyclic group, an aromatic carbocyclic group, an aromatic heterocyclic group or the like which is unsubstituted or substituted with one or more (for example, one, two, three, four, five or more) of the same or different substituents. Examples of acyl include, but are not limited to, acetyl (-COCH₃), benzoyl (-CO-Ph) and the like.

As used herein, the term "acyloxy" means -O-acyl, that is, -O-CO-R', where R' is as described in the item "acyl". Examples of acyloxy include, but are not limited to, acetoxy (-OCOCH₃), -OCO-n-C₁₅H₃₁, -OCO-n-C₁₇H₃₅ and the like.

As used herein, the term "carbonyl" means -(C=O)-.

As used herein, the term "oxo" means =O.

As used herein, the term "non-aromatic carbocycle" means a cyclic saturated hydrocarbon or a cyclic non-aromatic unsaturated hydrocarbon. A non-aromatic carbocycle may contain at least one double bond. A non-aromatic carbocyclic ring includes a monocyclic ring or fused rings such as bicyclic or tricyclic rings. One ring of a non-aromatic carbocycle is, for example, a 3 to 10 membered ring, typically, a 3 to 8 membered ring, a 3 to 6 membered ring, a 4 to 6 membered ring or a 5 to 6 membered ring. Examples of non-aromatic carbocycles include, but are not limited to, cycloalkane and cycloalkene, more specifically, cyclopropane, cyclobutane, cyclopentane, cyclopentene, cyclopentadiene, cyclohexane, cyclohexene and the like. A non-aromatic carbocycle may be substituted with one or more (for example, one, two, three, four, five or more) of the same or different substituents.

As used herein, the term "non-aromatic carbocyclic group" means a cyclic saturated hydrocarbon group or a cyclic non-aromatic unsaturated hydrocarbon group. A non-aromatic carbocyclic group is a monovalent group formed by removing a hydrogen atom from a non-aromatic carbocycle. A non-aromatic carbocyclic group may contain at least one double bond. Examples of non-aromatic carbocyclic groups include, but are not limited to, cycloalkyl and cycloalkenyl, specifically, cyclopentyl, cyclopentenyl, cyclopentadienyl, cyclohexyl, cyclohexenyl and the like. A non-aromatic carbocyclic group may be substituted with one or more (for example, one, two, three, four, five or more) of the same or different substituents.

As used herein, the term "non-aromatic heterocycle" means a non-aromatic ring containing one or more of the same or different heteroatoms optionally selected from O, S and N as a ring member. A non-aromatic heterocycle may contain at least one double bond. A non-aromatic heterocyclic ring includes a monocyclic ring or fused rings such as bicyclic or tricyclic rings. One ring of a non-aromatic heterocycle is, for example, a 3 to 10 membered ring, typically, a 3 to 8 membered ring, a 3 to 6 membered ring, a 4 to 6 membered ring or a 5 to 6 membered ring. Examples of non-aromatic heterocycles include, but are not limited to, tetrahydrofuran, dihydrofuran (2,5-dihydrofuran or 2,3-dihydrofuran), tetrahydropyran, dihydropyran (for example, 3,4-dihyro-2H-pyran), pyrrolidine, pyrroline, piperidine and the like. A non-aromatic heterocycle may be substituted with one or more (for example, one, two, three, four, five or more) of the same or different substituents. A non-aromatic heterocycle may include a lactone.

As used herein, the term "non-aromatic heterocyclic group" means a non-aromatic cyclic group containing one or more of the same or different heteroatoms optionally selected from O, S and N as a ring member. A non-aromatic heterocyclic group is a monovalent group formed by removing a hydrogen atom from a non-aromatic heterocycle. A non-aromatic heterocyclic group may contain at least one double bond. Examples of non-aromatic heterocyclic groups include, but are not limited to, tetrahydrofuranyl, dihydrofuranyl (2,5-dihydrofuranyl or 2,3-dihydrofuranyl), tetrahydropyranyl, dihydropyranyl (for example, 3,4-dihyro-2H-pyranyl), pyrrolidinyl, pyrrolinyl, piperidinyl and the like. A non-aromatic heterocyclic group may be substituted with one or more (for example, one, two, three, four, five or more) of the same or different substituents.

As used herein, the term "aromatic carbocycle" means a cyclic aromatic hydrocarbon. An aromatic carbocycle includes a monocyclic ring or fused rings such as bicyclic or tricyclic rings. One ring of an aromatic carbocycle is, for example, a 3 to 10 membered ring, typically, a 3 to 8 membered ring, a 3 to 6 membered ring, a 4 to 6 membered ring, a 5 to 6 membered ring or a 6 membered ring. Examples of aromatic carbocycles include, but are not limited to, benzene, naphthalene, anthracene and the like. An aromatic carbocycle may be substituted with one or more (for example, one, two, three, four, five or more) of the same or different substituents.

As used herein, the term "aromatic carbocyclic group" means a cyclic aromatic hydrocarbon group. An aromatic carbocyclic group is a monovalent group formed by removing a hydrogen atom from an aromatic carbocycle. Examples of aromatic carbocyclic groups include, but are not limited to, phenyl, naphthyl, anthracenyl and the like. An aromatic carbocyclic group may be substituted with one or more (for example, one, two, three, four, five or more) of the same or different substituents.

As used herein, the term "aromatic heterocycle" means an aromatic ring containing one or more of the same or different heteroatoms optionally selected from O, S and N as a ring member. An aromatic heterocycle includes a monocyclic ring or fused rings such as bicyclic or tricyclic rings. One ring of an aromatic heterocycle is, for example, a 3 to 10 membered ring, typically, a 3 to 8 membered ring, a 3 to 6 membered ring, a 4 to 6 membered ring or a 5 to 6 membered ring. Examples of aromatic heterocycles include, but are not limited to, furan, pyrrole, pyrazole, pyridine, pyridazine, pyrimidine, pyrazine, oxazole and the like. An aromatic heterocycle may be substituted with one or more (for example, one, two, three, four, five or more) of the same or different substituents.

As used herein, the term "aromatic heterocyclic group" means an aromatic cyclic group containing one or more of the same or different heteroatoms optionally selected from O, S and N as a ring member. An aromatic heterocyclic group is a monovalent group formed by removing a hydrogen atom from an aromatic heterocycle. Examples of aromatic heterocyclic groups include, but are not limited to, furanyl, pyrrolyl, pyrazolyl, pyridinyl, pyridazinyl, pyrimidinyl, pyrazinyl, oxazolyl and the like. An aromatic heterocyclic group may be substituted with one or more (for example, one, two, three, four, five or more) of the same or different substituents.

As used herein, the term "lactone" means a cyclic compound containing an ester group (-CO-O-) as ring members. A lactone may contain at least one double bond as ring members. The ring is, for example, a 3 membered ring, a 4 membered ring, a 5 membered ring, a 6 membered ring or a 7 membered ring. A lactone may be substituted with one or more (for example, one, two, three, four, five or more) of the same or different substituents.

As used herein, the term "substituent" means one or more (for example, one, two, three, four, five or more) groups that can be introduced in a given chemical structural formula. When two or more substituents are introduced, the substituents may be the same or different. Examples of substituents include, but are not limited to, alkyl, alkenyl, alkynyl, alkoxy, alkoxycarbonyl, hydroxy, hydroxyalkyl, carboxy, acyl, acyloxy, non-aromatic carbocyclic groups, non-aromatic heterocyclic groups, aromatic carbocyclic groups, aromatic heterocyclic groups, oxo and the like.

### (2. Agent for preventing decomposition of biopyrrin of the invention)

An agent for preventing decomposition of biopyrrin of the invention (also referred to as "agent for preventing decomposition of the invention") comprises an antioxidant substance having enediol structure; or a salt, an ester or a glycoside of an antioxidant substance having enediol structure; or a solvate, a stereoisomer or a tautomer of an antioxidant substance having enediol structure or a salt, an ester or a glycoside of an antioxidant substance having enediol structure. Enediol has a form of -C(OH)=C(OH)-. In some embodiments, an antioxidant substance having enediol structure can act as a reducing agent. An antioxidant substance having enediol structure may be a straight-chain compound or a cyclic compound.

Salts are, for example, sodium salts, potassium salts, calcium salts, magnesium salts or the like. Esters are, for example, esters formed with phosphoric acid, stearic acid, palmitic acid or the like. Esters can be carboxylic acid esters, phosphoric acid esters (for example, phosphoric acid monoesters), salts of phosphoric acid esters, for example, sodium salts, potassium salts, calcium salts, magnesium salts of phosphoric acid esters, or the like. Glycosides are, for example, O-glycosides that are formed with sugars such as glucose, galactose, mannose or fructose. The antioxidant substance having enediol structure may be stored in a form of salt, ester or glycoside thereof, and, immediately before use, it may be converted into the antioxidant substance having enediol structure such as artificially or by use of an enzyme in vivo. The salt, ester or glycoside may be formed with OH group in enediol structure. In this case, the salt, ester or glycoside may be formed with either one of the OH groups or both of the OH groups. When antioxidant substance having enediol structure has OH group(s) other than these of the enediol structure, the salt, ester or glycoside may be formed with the OH group(s) other than these of the enediol structure.

Agents for preventing decomposition of the invention can be provided as a single compound alone, a mixture of different kinds of compounds or a composition comprising a single compound or a mixture of different kinds of compounds. A composition can comprise excipients such as a solvent (for example, water, an alcohol or a mixture thereof).

Examples of antioxidant substances having enediol structure include, but are not limited to, compounds of formula (I) or (II), or salts, esters or glycosides thereof, or solvates, stereoisomers or tautomers thereof: 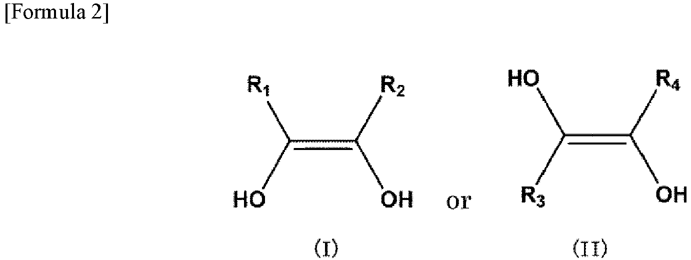 wherein R₁, R₂, R₃ and R₄ are, for example, each independently selected from a group consisting of hydrogen, alkyl, alkenyl, alkynyl, alkoxy, alkoxycarbonyl, hydroxy, hydroxyalkyl, carboxy, acyl, acyloxy, non-aromatic carbocyclic groups, non-aromatic heterocyclic groups, aromatic carbocyclic groups and aromatic heterocyclic groups. These alkyl, alkenyl, alkynyl, alkoxy, alkoxycarbonyl, hydroxy, hydroxyalkyl, acyl, acyloxy, non-aromatic carbocyclic groups, non-aromatic heterocyclic groups, aromatic carbocyclic groups and aromatic heterocyclic groups may be substituted with one or more (for example, one, two, three, four, five or more) of the same or different substituents.

Alternatively, R₁ and R₂ may form, with the carbon atoms to which they are attached, a ring such as a non-aromatic carbocycle, a non-aromatic heterocycle, an aromatic carbocycle or an aromatic heterocycle. The ring is, for example, a 3-membered ring, a 4-membered ring, a 5-membered ring, a 6-membered ring, a 7-membered ring or an 8-membered ring. The ring is, preferably, a 5-membered ring or a 6-membered ring. The non-aromatic heterocycle can include a lactone. The ring may be substituted with one or more (for example, one, two, three, four, five or more) of the same or different substituents. The ring may be a fused ring that consists of at least two or more of the same or different rings selected from a group consisting of non-aromatic carbocycles, non-aromatic heterocycles, aromatic carbocycles and aromatic heterocycles.

The substituents, preferably, are alkyl; alkyl substituted with one or more (for example, one, two, three, four, five or more) of the same or different substituents selected from a group consisting of hydroxy, carboxy, alkoxy, alkoxycarbonyl, oxo, acyl and acyloxy; or the like. The substituents are, preferably, hydroxyalkyl.

Further examples of antioxidant substances having enediol structure include, but are not limited to, reductones, or salts, esters or glycosides thereof, or solvates, stereoisomers or tautomers thereof, or the like. Specific examples of antioxidant substances having enediol structure include, but are not limited to, ascorbic acid, tartronic aldehyde, reductic acid, erythorbic acid, glucoreductones, α-pyridoin, α-pyridoin derivatives, catechins, or salts, esters or glycosides thereof, or solvates, stereoisomers or tautomers thereof, or the like.

Examples of reductones include, but are not limited to, compounds of the below formula (III) or (IV), or salts, esters or glycosides thereof, or solvates, stereoisomers or tautomers thereof, or the like: 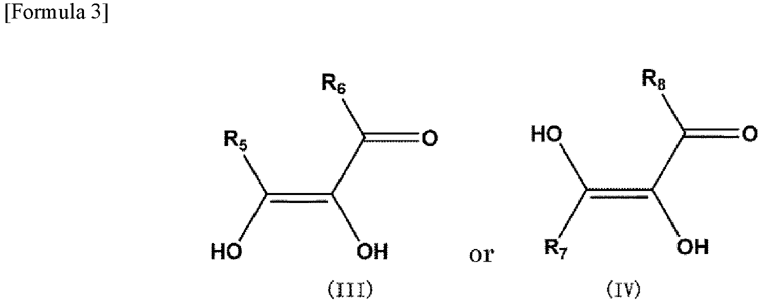 wherein R₅, R₆, R₇ and F₈ may be the same as those of R₁, R₂, R₃ and R₄ described in the above formula (I) or (II), respectively. Alternatively, R₅ and R₆ may be attached to form a ring such as a ring formed with R₁ and R₂ and the carbon atoms to which they are attached, as described in the above formula (I) or (II).

For example, in the formula, R₅, R₆, R₇ and R₈ are, each independently, selected from a group consisting of hydrogen, alkyl, alkenyl, alkynyl, alkoxy, alkoxycarbonyl, hydroxy, hydroxyalkyl, carboxy, acyl, acyloxy, non-aromatic carbocyclic groups, non-aromatic heterocyclic groups, aromatic carbocyclic groups and aromatic heterocyclic groups. These alkyl, alkenyl, alkynyl, alkoxy, alkoxycarbonyl, hydroxy, hydroxyalkyl, acyl, acyloxy, non-aromatic carbocyclic groups, non-aromatic heterocyclic groups, aromatic carbocyclic groups and aromatic heterocyclic groups may be substituted with one or more (for example, one, two, three, four, five or more) of the same or different substituents.

Alternatively, R₅ and R₆ may form, with the carbon atoms to which they are attached, a ring such as a non-aromatic carbocycle, a non-aromatic heterocycle, an aromatic carbocycle or an aromatic heterocycle. The ring is, for example, a 3-membered ring, a 4-membered ring, a 5-membered ring, a 6-membered ring, a 7-membered ring or an 8-membered ring. The ring is, preferably, a 5-membered ring or a 6-membered ring. The non-aromatic heterocycle can include a lactone. The ring may be substituted with one or more (for example, one, two, three, four, five or more) of the same or different substituents. The ring may be a fused ring that consists of at least two or more of the same or different rings selected from a group consisting of non-aromatic carbocycles, non-aromatic heterocycles, aromatic carbocycles and aromatic heterocycles.

The substituents, preferably, are alkyl; alkyl substituted with one or more (for example, one, two, three, four, five or more) of the same or different substituents selected from a group consisting of hydroxy, carboxy, alkoxy, alkoxycarbonyl, oxo, acyl and acyloxy; or the like. The substituents are, preferably, hydroxyalkyl.

Further examples of reductones include compounds having 3,4-dihydroxy-furan-2(5H)-one (which is also referred to as 3,4-dihydroxy-2(5H)-furanone) structure, compounds obtained by alkaline treatment of a sugar, or salts, esters or glycosides thereof, or solvates, stereoisomers or tautomers thereof. Specific examples of reductones include ascorbic acid, erythorbic acid, tartronic aldehyde, reductic acid, glucoreductones, or salts, esters or glycosides thereof, or solvates, stereoisomers or tautomers thereof, or the like.

The compounds having 3,4-dihydroxy-furan-2(5H)-one structure are, for example, compounds of the below formula (V), or salts, esters or glycosides thereof, or solvates, stereoisomers or tautomers thereof: 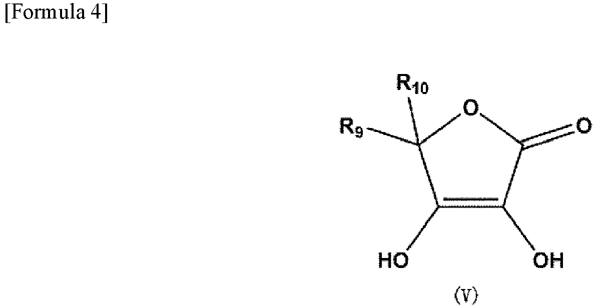 wherein R⁹ and R¹⁰ are, each independently, hydrogen; alkyl; or alkyl substituted with one or more (for example, one, two, three, four, five or more) of the same or different substituents selected from a group consisting of hydroxy, carboxy, alkoxy, alkoxycarbonyl, oxo, acyl, and acyloxy. Preferably, R⁹ is hydroxyalkyl, and R¹⁰ is hydrogen. More preferably, R⁹ is 1,2-dihydroxyethyl, and R¹⁰ is hydrogen.

Preferably, the compound of formula (V) is ascorbic acid, erythorbic acid, or a salt, an ester or a glycoside thereof, or a solvate, a stereoisomer or a tautomer thereof. Examples of the salts include, but are not limited to, sodium ascorbate, magnesium ascorbate, sodium erythorbate, magnesium erythorbate or the like. Examples of the esters include, but are not limited to, ascorbyl palmitate, ascorbyl stearate, sodium ascorbyl phosphate, magnesium ascorbyl phosphate, erythorbyl palmitate, erythorbyl stearate, sodium erythorbyl phosphate, magnesium erythorbyl phosphate or the like. Examples of the glycosides include, but are not limited to, ascorbic acid 2-glucoside, ascorbic acid 6-glucoside, erythorbic acid 2-glucoside, erythorbic acid 6-glucoside or the like. Ascorbic acid or erythorbic acid can include L-form, D-form, or a mixture thereof with any ratio (for example, 1:1, 1:2, 1:3, 1:4, 1:5, 2:1, 3:1, 4:1 or 5:1).

The antioxidant substances having enediol structure, including ascorbic acid, or salts, esters or glycosides thereof, or solvates, stereoisomers or tautomers thereof are commercially available. Alternatively, a person skilled in the art can prepare the antioxidant substances having enediol structure, or salts, esters or glycosides thereof, or solvates, stereoisomers or tautomers thereof on the basis of general synthetic methodology in organic chemistry.

### (3. Methods of using the agent for preventing decomposition of biopyrrin of the invention)

The agent for preventing decomposition according to the invention can be used for preventing decomposition of biopyrrin, particularly, for preventing decomposition of biopyrrin in a biological sample. The agent for preventing decomposition of the invention is added to a biological sample to prevent the decomposition of biopyrrin in the biological sample. Thereby, a biological sample to which the agent for preventing decomposition of the invention is added, a biological sample comprising the agent for preventing decomposition of the invention, and a mixture of the agent for preventing decomposition of the invention and a biological sample are provided. Biological samples to which the agent for preventing decomposition of the invention is added can prevent the decomposition of biopyrrin at ambient temperature or lower for a long term, and therefore, can be stored for a long term.

In one embodiment, the agent for preventing decomposition of the invention is used in a method of preventing decomposition of biopyrrin in a biological sample. The method comprises a step of adding the agent for preventing decomposition of the invention to the biological sample. Furthermore, the agent for preventing decomposition of the invention can be used in a method of storing a biological sample. The method comprises steps of:
(a) adding the agent for preventing decomposition of the invention to the biological sample; and
(b) storing the biological sample at ambient temperature or lower.

The agent for preventing decomposition of the invention does not affect measurements of biopyrrin in a biological sample. Accordingly, in other embodiments, the agent for preventing decomposition of the invention is used in a method of measuring a concentration of biopyrrin in a biological sample. The method comprises steps of:
(a) adding the agent for preventing decomposition of the invention to the biological sample; and
(b) measuring the concentration of biopyrrin in the biological sample.

The method can further comprise a step of storing the biological sample at ambient temperature or lower after step (a) and before step (b).

The concentration of biopyrrin in a urine sample may change depending on a degree of condensation of the urine sample. Therefore, in general, creatinine is used as an internal standard, and a concentration of biopyrrin is provided as a relative amount to a concentration of creatinine. Accordingly, in general, the concentration of creatinine is also measured at the same time when the concentration of biopyrrin is measured. The agent for preventing decomposition of the invention does not affect the measurement of a concentration of creatinine in a biological sample. Therefore, in one embodiment, the agent for preventing decomposition of the invention is used in a method of measuring concentrations of biopyrrin and creatinine in the same biological sample. In this case, the concentration of biopyrrin can be provided as a relative amount to the concentration of creatinine as an internal standard. The method comprises steps of:
(a) adding the agent for preventing decomposition of the invention to the biological sample; and
(b) measuring the concentration of biopyrrin and the concentration of creatinine in the biological sample.

The method can further comprise a step of calculating a relative value of the concentration of biopyrrin to the concentration of creatinine. Either the concentration of biopyrrin or the concentration of creatinine could be measured firstly. The method can further comprise a step of storing the biological sample at ambient temperature or lower after step (a) and before step (b).

Biopyrrin is known as an oxidative stress marker, a marker for psychiatric disorders and a marker for at-risk mental state (ARMS). Therefore, in one embodiment, the agent for preventing decomposition of the invention is used in a method of assessing an oxidative stress state in a subject. The method comprises steps of:
(a) adding the agent for preventing decomposition of the invention to a biological sample obtained from the subject; and
(b) measuring a concentration of biopyrrin in the biological sample.

The method can further comprise a step of storing the biological sample at ambient temperature or lower after step (a) and before step (b). When the concentration measured in step (b) is higher than a concentration of biopyrrin in a reference sample (for example, about 1.2 times, about 1.5 times, about 2 times, about 3 times, about 5 times, about 10 times higher or more), it can indicate that the subject is under the oxidative stress state. The reference sample may be a biological sample from a healthy subject, a biological sample obtained from the subject oneself in the past (for example, when the subject was in good health), or the like.

In other embodiments, the agent for preventing decomposition of the invention is used in a method of diagnosing diseases caused by oxidative stress (for example, psychiatric disorders) or ARMS in a subject or a method of obtaining data for diagnosing these states. The diagnosis can comprise identifying a subject with diseases caused by oxidative stress or ARMS, and/or determining a disease stage of the subject. The method comprises steps of:
(a) adding the agent for preventing decomposition of the invention to a biological sample obtained from the subject; and
(b) measuring a concentration of biopyrrin in the biological sample.

The method can further comprise a step of storing the biological sample at ambient temperature or lower after step (a) and before step (b). When the concentration measured in step (b) is higher than a concentration of biopyrrin in a reference sample (for example, about 1.2 times, about 1.5 times, about 2 times, about 3 times, about 5 times, about 10 times higher or more), it can indicate that there is a possibility that the subject has a disease caused by oxidative stress or the subject is ARMS. When the concentration measured in step (b) is higher than a concentration of biopyrrin in a reference sample (for example, about 1.2 times, about 1.5 times, about 2 times, about 3 times, about 5 times, about 10 times higher or more), it may indicate that the disease of the subject is progressing in a clinical stage. The reference sample may be a biological sample from a healthy subject, a biological sample obtained from the subject oneself in the past (for example, when the subject was in good health), a biological sample from a subject in a certain clinical stage, or the like.

In further other embodiments, the agent for preventing decomposition of the invention is used in a method of assessing the prognosis of oxidative stress states, diseases caused by oxidative stress (for example, psychiatric disorders) or ARMS in subjects after drug treatment, or in a method of obtaining data for assessing the same. In addition, the agent for preventing decomposition of the invention is used in a method of screening candidate drugs for the treatment or prevention of oxidative stress states, diseases caused by oxidative stress (for example, psychiatric disorders) or ARMS, or in a method of obtaining data for screening the same. The method comprises steps of:
(a) adding the agent for preventing decomposition of the invention to a biological sample obtained from a subject before administering a drug;
(b) measuring the first concentration of biopyrrin in the biological sample;
(c) adding the agent for preventing decomposition of the invention to a biological sample obtained from the subject after administering the drug; and
(d) measuring the second concentration of biopyrrin in the biological sample.

The method can further comprise a step of storing the biological sample at ambient temperature or lower after step (a) and before step (b), and/or, after step (c) and before step (d). When the second concentration is lower than the first concentration (for example, about 5/6, about 3/2, about 1/2, about 1/3, about 1/5, about 1/10 or less), it may indicate that the oxidative stress state, disease caused by oxidative stress or ARMS is improved by the drug, and the prognosis is favorable. When the second concentration is lower than the first concentration (for example, about 5/6, about 3/2, about 1/2, about 1/3, about 1/5, about 1/10 or less), the drug may be a candidate drug for the treatment or prevention of oxidative stress state, diseases caused by oxidative stress or ARMS. The candidate drug for the treatment or prevention obtained by such screening method may be used in a method for the treatment or prevention of oxidative stress state, diseases caused by oxidative stress or ARMS in a subject. The method for the treatment or prevention comprises administrating the candidate drug for the treatment or prevention to a subject. The candidate drug for the treatment or prevention obtained by such screening method may be used in a method of preparing a pharmaceutical composition for the treatment or prevention of oxidative stress state, diseases caused by oxidative stress or ARMS. The pharmaceutical composition for the treatment or prevention comprises the candidate drug for the treatment or prevention. The pharmaceutical composition can further comprise any pharmaceutically acceptable excipients such as a solvent and the like.

The diseases caused by oxidative stress (for example, psychiatric disorders) includes, but are not limited to, schizophrenia, autism, Alzheimer's disease, cognitive abnormality, depression, bipolar disorder (manic-depressive psychosis), neurodevelopmental disorder with anomalies of the brain, cognitive impairment ascribed to neuropathy due to infection during pregnancy, mental disorder ascribed to impaired immunity, epilepsy, idiophrenic insanity, toxic psychosis, intellectual disability (mental retardation), psychopathy, neurosis, psychiatric disorder ascribed to syphilitic infection, senile psychiatric disorders, cerebrovascular disorders, psychiatric disorders due to a head injury, atypical endogenous psychosis, endocrine and extrinsic psychiatric disorders, involutional psychosis, and the like.

Specific methods for applying the agent for preventing decomposition of the invention to a biological sample are further described below. The following description can apply to any of the above-mentioned methods.

The agent for preventing decomposition of the invention is added to a biological sample. After the addition, the biological sample may further be mixed. The method of adding the agent for preventing decomposition of the invention to a biological sample is not limited. For example, the agent for preventing decomposition of the invention is added using a pipette, a micropipette, a syringe, or the like. The optimal timing for the addition of the agent for preventing decomposition of the invention to a biological sample should be at the time immediately after obtaining the biological sample from a subject. For example, the addition time is within about 1 second, about 5 seconds, about 10 seconds, about 30 seconds, or about 60 seconds after obtaining a biological sample from a subject. Preferably, the agent for preventing decomposition of the invention is added immediately after collecting a biological sample from a subject. It is assumed that the decomposition of biopyrrin starts immediately after collecting a sample, thus it is preferable to add the agent for preventing decomposition of the invention to the sample, even if the sample is not intended to be stored for long-term.

The agent for preventing decomposition of the invention is added so that the final concentration of the antioxidant substance having enediol structure is, for example, about 0.1 mM to about 50 mM, about 0.5 mM to about 30 mM, about 1 mM to about 20 mM, about 2 mM to 20 mM, about 3 mM to about 20 mM, about 4 mM to about 20 mM, about 5 mM to about 15 mM, about 5 mM to about 10 mM, preferably, about 10 mM. The final concentration of the antioxidant substance having enediol structure can vary depending on the type of the biological sample, the predicted amount of biopyrrin in the biological sample, a storage method, a storage period and the like.

The biological sample is collected from a subject. The collection method may be a common method known by a person skilled in the art, and is not particularly limited. The subject includes humans or non-human animals. The non-human animals include, for example, non-human mammals such as rats, mice, guinea pigs, rabbits, monkeys, dogs, cats, miniature pigs or the like. When the subject is a non-human animal, the subject may be a male or a female (pregnant or non-pregnant). When the subject is a human, the subject may be also referred to as "examinee" or "patient". When the subject is a human, the subject may be a male or a female (pregnant or non-pregnant). The age stages of the subject may include, but are not limited to, a neonate, suckling infant, infant, juvenile (boy or girl), sub-adult (young-adult), prime-age adult, middle-aged adult, or older adult.

The types of biological samples include, but are not limited to, urine, blood (serum, plasma or whole blood), tissues, cells or the like. Preferably, the biological sample is urine. The biological sample, if desired, may be subjected to pre-treatments such as homogenization, pulverization, separation (e.g. centrifugation), filtration, extraction, the addition of solvents and/or the addition of surfactants and/or the like. For example, in the case that the biological sample is urine, it can be directly measured without pre-treatments. However, in the case that the biological sample is whole blood and the measurement of the sample provides a colorimetric result, the result may be interfered by red blood cells and be indistinguishable. Therefore, it is preferable to separate the whole blood in that case. The biological sample may be pre-treated for the measurements after adding the agent for preventing decomposition of the invention to the biological sample. The biological sample may contain biopyrrin, but the agent for preventing decomposition of the invention can be used for a biological sample containing biopyrrin, biological sample that is known to contain biopyrrin, biological sample that may contain biopyrrin, and biological sample that is unknown whether or not it contains biopyrrin.

The concentration of biopyrrin in a biological sample can be measured by a common method known to a person skilled in the art. For example, the measurement can be conducted by use of HPLC, mass spectrometry, ELISA and immune chromatography. Particularly, the measurements can be conducted using Biopyrrin ELISA kit, Redox Assay (Metallogenics Co., Ltd). The concentration of creatinine in a biological sample can be also measured by a common method known to a person skilled in the art. For example, the measurement can be conducted by use of HPLC, mass spectrometry, ELISA and immune chromatography. Particularly, the measurement can be conducted using LabAssay Creatinine (FUJIFILM Wako Pure Chemical Corporation).

A biological sample to which the agent for preventing decomposition of the invention is added can prevent the decomposition of biopyrrin at ambient temperature or lower for a long term. Accordingly, a biological sample to which the agent for preventing decomposition of the invention is added can be stored for a long term. The ambient temperature or lower is, for example, a room temperature (e.g. about 10°C to about 40°C, about 15°C to about 35°C, about 20°C to about 30°C or about 25°C to about 27°C) or under low temperature conditions (e.g. a temperature in a refrigerator, particularly, about 0°C to about 10°C, about 0°C to about 8°C, about 2°C to about 6°C or about 2°C to about 4°C, or a temperature in a freezer, particularly, about -80°C or below, about -80°C to about -0°C, about -60°C to about -0°C, about -40°C to about -0°C, about -30°C to about -0°C, about -20°C to about -10°C, about -15°C to about -20°C or about - 18°C). Preferably, the temperature is a room temperature or about 4°C. The storage period is about 0.5 hours to about 240 hours, about 1 hour to about 120 hours, for example, about 30 minutes, about 1 hour, about 3 hours, about 6 hours, about 12 hours, about 24 hours (about 1 day), about 36 hours, about 48 hours (about 2 days), about 60 hours, about 72 hours (about 3 days), about 84 hours, about 96 hours (about 4 days), about 108 hours, about 120 hours (about 5 days), about 144 hours (about 6 days), about 168 hours (about 7 days), about 192 hours (about 8 days), about 216 hours (about 9 days) or about 240 hours (about 10 days), or more.

Preferably, a biological sample to which the agent for preventing decomposition of the invention is added, is stored at a room temperature for about 24 hours (about 1 day), about 48 hours (about 2 days) or about 72 hours (about 3 days), or more. Preferably, a biological sample to which the agent for preventing decomposition of the invention is added, is stored at a low temperature for about 72 hours (about 3 days), about 96 hours (about 4 days) or about 120 hours (about 5 days), or more.

### Examples

### (4. Examples)

Examples of the present invention will be described below. The following Examples are described for a better understanding of the scope of claims of the present invention, and are not intended to limit the scope of claims of the present invention. In the following Examples, "ascorbic acid" means L-ascorbic acid.

### (Example 1: Investigation of agents for preventing decomposition of biopyrrin)

It was investigated by adding an antioxidant substance to a urine sample whether or not the decomposition of biopyrrin in the urine sample could be prevented. Ascorbic acid (FUJIFILM Wako Pure Chemical Corporation), sodium chloride (FUJIFILM Wako Pure Chemical Corporation), reduced glutathione (NACALAI TESQUE, INC.), L-cysteine (FUJIFILM Wako Pure Chemical Corporation), riboflavin (FUJIFILM Wako Pure Chemical Corporation) and peroxidase (FUJIFILM Wako Pure Chemical Corporation) were selected as antioxidant substances.

Urine samples were collected from humans (N=4), an antioxidant substance was added to each of the urine samples, and they were incubated for 48 hours at 27°C. Then, each of the concentrations of biopyrrin in the samples was measured. In preparing a standard sample, a urine sample collected from each of the humans was stored at -80°C without addition of any antioxidant substances. Each of biopyrrin concentrations in urine samples was measured by Biopyrrin ELISA kit, Redox Assay (Metallogenics Co., Ltd).

The results are shown in Figure 1 and Table 1. The concentrations of the antioxidant substances in the table mean the final concentrations. Each measured values was calibrated with the concentration of biopyrrin in the standard sample (antioxidant substance-free, stored at -80°C) as 100%. When ascorbic acid was used as an antioxidant substance, the concentration of biopyrrin was almost the same as that of the standard sample. This result indicates that ascorbic acid can be used as an agent for preventing decomposition of biopyrrin.

**[Table 1]**

| Antioxidant Substance | Relative Concentration of Biopyrrin |
|---|---|
| Ascorbic acid 10 mM | 100 ± 3% |
| Ascorbic acid 3 mM | 100 ± 5% |
| Sodium Chloride | 47.3 ± 5% |
| Glutathione 0.1 mM | 46.8 ± 5% |
| Glutathione 0.03 mM | 51.9 ± 11% |
| Riboflavin 0.1 mM | 35.5 ± 6% |
| Riboflavin 0.03 mM | 17.7 ± 1% |
| Peroxidase 1 ug/ml | Undetectable |
| Peroxidase 0.3 ug/ml | Undetectable |
| L-Cysteine 0.1 mM | 53.3 ± 7% |
| L-Cysteine 0.03 mM | 49.8 ± 6% |
| No antioxidant treatment | 49.8 ± 6% |

Without being bound by theory, ascorbic acid can scavenge more than one type of reactive oxygen species (O²⁻, H₂O₂, ¹O₂), superior to other antioxidant substances. It is known that this superior antioxidant effect of ascorbic acid is due to the enediol structure of ascorbic acid (for example, see Journal of Nippon Medical School, 2013:9(3), in particular, Fig.3 on p.167 and the second paragraph of the left column on p.168.). Considering each of chemical structures of the antioxidant substances used in the Examples, it is considered that it is important to have enediol structure in preventing decomposition of biopyrrin. Accordingly, it is considered that antioxidant substances having enediol structure other than ascorbic acid, would also have a function of preventing decomposition of biopyrrin.

### (Example 2: Chronological change in concentration of biopyrrin in urine sample under severe temperature conditions)

In this experiment, we investigated whether or not the agent for preventing decomposition of the invention can prevent the decomposition of biopyrrin in a urine sample under severe temperature conditions during midsummer (about 28°C to about 32°C).

Urine samples were collected from three healthy subjects (two males and one female), and the samples were stored for 5 days under each of the following conditions (1) to (3). In preparing a standard sample, a urine sample collected from each of the humans was stored at -80°C without addition of any antioxidant substances. Each of biopyrrin concentrations in urine samples was measured at the time of 24 hours, 48 hours, 72 hours, 96 hours and 120 hours after the start of the experiment. The measurements were conducted by Biopyrrin ELISA kit (Metallogenics Co., Ltd).

### (Condition)

(1) No antioxidant treatment: stored at 28°C to 32°C
(2) No antioxidant treatment: stored at 4°C
(3) Added with ascorbic acid (10 mM) immediately after collecting a urine sample, and stored at 28°C to 32°C.

The results are shown in Table 2. The concentrations of the antioxidant substances in the table mean the final concentrations. Each measured values was calibrated with the concentration of biopyrrin in the standard sample (antioxidant substance-free, stored at -80°C) as 100%. As shown in Table 2, it has been found that ascorbic acid can prevent decomposition of biopyrrin even under severe conditions.

**[Table 2]**

| | No antioxidant treatment (28 to 32°C) | No antioxidant treatment (4°C) | Ascorbic acid 10 mM added (28 to 32°C) |
|---|---|---|---|
| 24 hours | 58 ± 20% | 85.4 ± 2.8% | 95.8 ± 7.4% |
| 48 hours | 33.6 ± 13.1% | 74.6 ± 3.7% | 98.1 ± 7.5% |
| 72 hours | 32.7 ± 23.6% | 66.1 ± 9.8% | 98.4 ± 6.2% |
| 96 hours | 21.6 ± 17% | 48.9 ± 12.3% | 84.4 ± 5% |
| 120 hours | 21.4 ± 12.5% | 42.6 ± 10.3% | 88.3 ± 8.7% |

| | | | |
|---|---|---|---|
| Mean ± Standard Error N=3 | | | |

### (Example 3: Chronological changes in concentration of biopyrrin in urine sample under low temperature conditions)

In this experiment, we investigated to what degree the agent for preventing decomposition of the invention can prevent the decomposition of biopyrrin under low temperature conditions (approximately 4°C).

The same urine samples as examined in Example 2 were used. Ascorbic acid was added (final concentration; 10 mM) into each of the samples, and the samples were stored at 4°C. The concentrations of biopyrrin in the samples were measured at the time of 24 hours, 48 hours, 72 hours, 96 hours and 120 hours after the start of the experiment. The measurements were conducted by Biopyrrin ELISA kit (Metallogenics Co., Ltd).

The results are shown in Table 3 and Figure 2. The concentrations of biopyrrin were not changed compared with the case stored at -80°C. These results indicate that a urine sample can be stored stably for 120 hours or more when the sample is stored at 4°C by using 10 mM of ascorbic acid.

**[Table 3]**

| Hours | Biopyrrin concentration (umol/L) |
|---|---|
| - (Case stored at -80°C) | 8.5 |
| 24 | 8.7 |
| 48 | 8.9 |
| 72 | 8.3 |
| 96 | 8.6 |
| 120 | 8.7 |

### (Example 4: Confirmation of the influence on creatinine)

The concentration of biopyrrin in a urine sample may change depending on a degree of condensation of the urine sample. Therefore, in general, creatinine is used as an internal standard, and the concentration of biopyrrin is provided as a relative amount to the concentration of creatinine. If an antioxidant substance affects the concentration or measurement of creatinine, the relative amount of biopyrrin cannot be accurately measured. Therefore, it was investigated whether or not the antioxidant substance of the invention affects the measurement of the creatinine concentration.

Urine samples were collected from humans (N=4), ascorbic acid was added to each of the samples, and they were incubated for 48 hours at 27°C. Then, each of the concentrations of creatinine in the samples was measured. In preparing a standard sample, a urine sample collected from each of the humans was stored at -80°C without addition of any antioxidant substances. Each of creatinine concentrations in urine samples was measured by LabAssay Creatinine (FUJIFILM Wako Pure Chemical Corporation).

The results are shown in Table 4. The concentrations of the antioxidant substances in the table mean the final concentrations. Each measured values was calibrated with the concentration of each ingredients in the standard sample (antioxidant substance-free, stored at -80°C) as 100%. It has been found that ascorbic acid does not affect the measurement of the creatinine concentration.

**[Table 4]**

| Antioxidant Substance | Relative Concentration of Creatinine |
|---|---|
| Ascorbic acid 10 mM | 94.6 ± 5% |
| Ascorbic acid 3 mM | 98.3 ± 5% |
| No antioxidant treatment | 92.7 ± 10% |

In addition to the above, experiments under the same conditions as in Example 2 and measurement of the concentration of creatinine in the same manner as above in this Example were conducted. The results are shown in Table 5. The concentrations of the antioxidant substance in the table mean final concentrations. Each measured value was calibrated with the concentration of the ingredient in the standard sample (antioxidant substance-free, stored at -80°C) as 100%. It has been found that ascorbic acid does not affect the measurement of the creatinine concentration even under severe conditions of 28°C to 32°C.

**[Table 5]**

| Relative Concentration of Creatinine | | | | |
|---|---|---|---|---|
| | No antioxidant treatment (28 to 32°C) | No antioxidant treatment (4°C) | Ascorbic acid 5 mM added (28 to 32°C) | Ascorbic acid 10 mM added (28 to 32°C) |
| 24 hours | 103 ± 1% | 101 ± 1% | 106 ± 1% | 99.2 ± 0.9% |
| 48 hours | 102 ± 2% | 106 ± 3% | 102 ± 0.6% | 104 ± 0.9% |
| 72 hours | 94 ± 2% | 105 ± 3.5% | 99.1 ± 0.8% | 104 ± 2.9% |
| 96 hours | 95 ± 3% | 102 ± 2.4% | 94.9 ± 2.7% | 103 ± 3.1% |
| 120 hours | 101 ± 3% | 102 ± 2.5% | 102 ± 2.5% | 103 ± 1.8% |

| | | | | |
|---|---|---|---|---|
| Mean ± Standard Error N=3 | | | | |

## Claims

1. An agent for preventing decomposition of biopyrrin, comprising an antioxidant substance having enediol structure, or a salt, an ester or a glycoside thereof, or a solvate, a stereoisomer or a tautomer thereof.

2. The agent for preventing decomposition according to claim 1, wherein the antioxidant substance having enediol structure is a reductone.

3. The agent for preventing decomposition according to claim 2, wherein the reductone is a compound having 3,4-dihydroxy-furan-2(5H)-one structure.

4. The agent for preventing decomposition according to claim 3, wherein the compound having 3,4-dihydroxy-furan-2(5H)-one structure is a compound of formula (V): 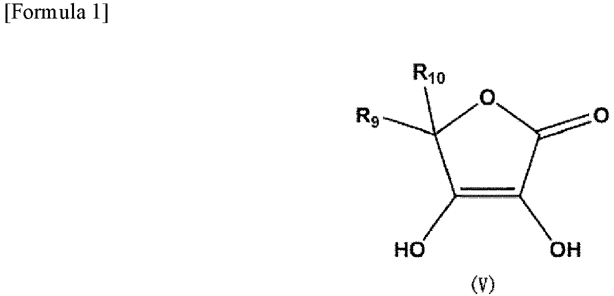 wherein R⁹ and R¹⁰ are each independently hydrogen; alkyl; or alkyl substituted with one or more of the same or different substituents selected from a group consisting of hydroxy, carboxy, alkoxy, alkoxycarbonyl, oxo, acyl and acyloxy.

5. The agent for preventing decomposition according to claim 4, wherein the compound of formula (V) is ascorbic acid.

6. A biological sample to which added is the agent for preventing decomposition according to any one of claims 1 to 5.

7. The biological sample according to claim 6, wherein the biological sample is urine.

8. The biological sample according to claim 6 or 7, wherein the antioxidant substance having enediol structure comprised in the agent for preventing decomposition is present in the biological sample at a concentration within a range of about 1 mM to about 20 mM.

9. The biological sample according to any one of claims 6 to 8, wherein the antioxidant substance having enediol structure comprised in the agent for preventing decomposition is present in the biological sample at a concentration of about 10 mM.

10. A method of preventing decomposition of biopyrrin in a biological sample, comprising a step of adding the agent for preventing decomposition according to any one of claims 1 to 5 to the biological sample.

11. A method of storing a biological sample, comprising steps of:
(a) adding the agent for preventing decomposition according to any one of claims 1 to 5 to the biological sample; and
(b) storing the biological sample at ambient temperature or lower.

12. A method of measuring a concentration of biopyrrin in a biological sample, comprising steps of:
(a) adding the agent for preventing decomposition according to any one of claims 1 to 5 to the biological sample; and
(b) measuring the concentration of biopyrrin in the biological sample.

13. The method according to claim 12, further comprising a step of storing the biological sample at ambient temperature or lower after step (a).

14. A method of obtaining data for assessing an oxidative stress state in a subject, comprising steps of:
(a) adding the agent for preventing decomposition according to any one of claims 1 to 5 to a biological sample obtained from the subject; and
(b) measuring a concentration of biopyrrin in the biological sample;
wherein, when the measured concentration is higher than a concentration of biopyrrin in a reference sample, the concentration data indicates that the subject is under the oxidative stress state.

15. The method according to claim 14, further comprising a step of storing the biological sample at ambient temperature or lower after step (a).

16. The method according to any one of claims 10 to 15, wherein the biological sample is urine.

17. The method according to any one of claims 10 to 16, wherein the antioxidant substance having enediol structure comprised in the agent for preventing decomposition is added to a concentration within a range of about 1 mM to about 20 mM.

18. The method according to any one of claims 10 to 17, wherein the antioxidant substance having enediol structure comprised in the agent for preventing decomposition is added to a concentration of about 10 mM.
